# EUROPEAN PATENT APPLICATION

(11) **EP 1 925 335 A1**
(43) Date of publication of application: **28.05.2008**
(21) Application number: 06797957.5
(22) Date of filing: 14.09.2006
(51) Int. Cl.: A61N 1/30, A61M 25/00

(54) **CATHETER TYPE IONTOPHORESIS APPARATUS**

(30) Priority: 16.09.2005 JP 2005270862
(71) Applicant: TTI ELLEBEAU, INC., Tokyo 140-0002 (JP)
(72) Inventor: AKIYAMA, Hidero, Higashi-shinagawa, Shinagawa-ku Tokyo 140-0002 (JP); KAWAKAMI, Hiroyoshi, Tokyo 192-0373 (JP); NAKAYAMA, Mizuo, Higashi-shinagawa, Shinagawa-ku Tokyo 140-0002 (JP); MATSUMURA, Takehiko, Higashi-shinagawa, Shinagawa-ku Tokyo 140-0002 (JP); MATSUMURA, Akihiko, Higashi-shinagawa, Shinagawa-ku Tokyo 140-0002 (JP)
(74) Representative: Geyer, Ulrich F.
(86) International application number: PCT/JP2006/318239
(87) International publication number: WO 2007/032423

(57) **Abstract**

A catheter-type iontophoresis device (10) includes a small working side electrode assembly (12) and a small non-working side electrode assembly (14) each arranged at the tip of a flexible cable 18 of an endoscopic device (20). A first ion exchange membrane 44 and a fourth ion exchange membrane (54) at the tips of the assemblies are brought into close contact with, for example, a cancer site of a digestive organ so that a drug solution is caused to permeate on a pinpoint basis by means of iontophoresis. Each of the working side electrode assembly (12) and the non-working side electrode assembly (14) is attached to the tip of a rod-like member (16). The rod-like member 16 is detachable from the tip of the flexible cable 18, and is hence exchangeable integrally with the cable.

## Description

### Technical Field

The present invention relates to an iontophoresis device for administering a drug ion to an organism.

### Background Art

Such iontophoresis device as described above is intended for causing a drug solution to permeate into a skin or a mucosa, and its object has been conventionally a skin or mucosa having a relatively large area of at least about 20 mm in diameter.

On the other hand, the direct injection of a drug into: a region as an object of endoscopic surgery; part of a region such as a mucosa in a nasal cavity, a mucosa in an oral cavity, an esophageal, a stomach, a small intestine, a large intestine, or an anal; an affected area upon laparoscopic operation in a lung cancer therapy; or part of an organism exposed in laparotomy or the like (pinpoint) may increase a therapeutic effect.

In such case, the permeation of a drug by means of iontophoresis rather than injection is non-invasive and preferable.

In addition, upon photodynamic therapy (PDT), after a photosensitizing reaction substance has been administered, light is applied so that an anti-cancer action can be expected. However, a patient must not be irradiated with sunlight because the photosensitizing reaction substance circulates through his or her body. In addition, the substance may circulate through any portion other than an affected area to provide a side effect. Therefore, in a PDT, the administration of a photosensitizing reaction substance to only an affected area has been desired.

### Disclosure of the Invention

An object to be achieved by the present invention is to provide an iontophoresis device suitably used upon permeation of a drug solution into part of an organism such as a cancer site in case of a therapy or treatment by means of an endoscope or a laparoscope.

The above-described objectives are achieved by the following embodiments of the present invention.

(1) A catheter-type iontophoresis device including a working side electrode assembly and a non-working side electrode assembly each used for administering an ionic drug by iontophoresis and a DC electric power source to be connected to the working side electrode assembly and the non-working side electrode assembly with opposite polarities, characterized by including: a rod-like member for supporting the working side electrode assembly and the non-working side electrode assembly; and an endoscope device for detachably supporting the rod-like member, the working side electrode assembly and the non-working side electrode assembly being disposed at a tip of the rod-like member, a predetermined amount of spacing being provided between the working side electrode assembly and the non-working side electrode assembly, and the rod-like member being detachably supported at a tip of a flexible cable flexibly supported by a flexible tube of the endoscope device.

(2) The catheter-type iontophoresis device according to item (1), characterized in that the ionic drug is a photosensitized reactive material to be activated by absorbing light, and the endoscope device has an irradiation optical system for applying light from a neighborhood of a tip of the working side electrode assembly via the flexible tube.

(3) The catheter-type iontophoresis device according to item (2), characterized in that the endoscope device has an endoscope optical system including an optical fiber for irradiated light for irradiating an inside of an organism with light and an optical fiber for reflected light for introducing reflected irradiated light to an outside, and the irradiation optical system is the optical fiber for irradiated light.

(4) The catheter-type iontophoresis device according to any one of items (1) to (3), characterized in that the flexible cable includes an electric power source side working electrode terminal and an electric power source side non-working electrode terminal connected via wiring from the DC electric power source to the DC electric power source with opposite polarities, the wiring being housed in the flexible cable, the rod-like member has on a proximal end of a side thereof detachable from the flexible cable a working electrode side contact and a non-working electrode side contact which are connected to or are separated from the electric power source side working electrode terminal and the electric power source side non-working electrode terminal when attached to or detached from the flexible cable, and the working electrode side contact and the non-working electrode side contact are connected to a working side electrode and a non-working side electrode in the working side electrode assembly and the non-working side electrode assembly, respectively.

(5) The catheter-type iontophoresis device according to item (4), characterized in that the endoscope device includes a controller for adjusting at least a current value out of a current value during energization and energization time as administration time, the controller being disposed in an electric power source circuit between each of the electric power source side working electrode terminal and the electric power source side non-working electrode terminal and the DC electric power source.

(6) The catheter-type iontophoresis device according to any one of items (1) to (5), characterized in that the working side electrode assembly and the non-working side electrode assembly are disposed such that central axes thereof are in parallel to each other.

(7) The catheter-type iontophoresis device according to any one of items (1) to (5), characterized in that the working side electrode assembly and the non-working side electrode assembly are disposed such that central axes thereof spread in a tip direction.

(8) The catheter-type iontophoresis device according to any one of items (1) to (5), characterized in that the working side electrode assembly and the non-working side electrode assembly are disposed such that central axes thereof intersect each other in a tip direction.

(9) The catheter-type iontophoresis device according to any one of items (1) to (8), characterized in that the working side electrode assembly includes: the working side electrode connected to the DC electric power source having the same polarity as that of a charged ion of the ionic drug; an electrolyte solution holding portion holding an electrolyte solution, the electrolyte solution holding portion being disposed on a front surface of the working electrode; a second ion exchange membrane selecting an ion having a polarity opposite to that of the charged ion of the ionic drug, the second ion exchange membrane being disposed on a front surface of the electrolyte solution holding portion; a drug solution holding portion holding the ionic drug, the drug solution holding portion being disposed on a front surface of the second ion exchange membrane; and a first ion exchange membrane which is the ion exchange membrane selecting an ion having the same polarity as that of the charged ion of the ionic drug, the first ion exchange membrane being disposed on a front surface of the drug solution holding portion, and the non-working side electrode assembly includes: the non-working side electrode connected to the DC electric power source with a polarity opposite to that of the charged ion of the ionic drug; a second electrolyte solution holding portion holding a second electrolyte solution, the second electrolyte solution holding portion being disposed on a front surface of the non-working side electrode; a third ion exchange membrane selecting an ion having a polarity same to that of the charged ion of the ionic drug, the third ion exchange membrane being disposed on a front surface of the second electrolyte solution holding portion; a third electrolyte solution holding portion holding a third electrolyte solution, the third electrolyte solution holding portion being disposed on a front surface of the third ion exchange membrane; and a fourth ion exchange membrane which is the ion exchange membrane selecting an ion having a polarity opposite to that of the charged ion of the ionic drug, the fourth ion exchange membrane being disposed on a front surface of the third electrolyte solution holding portion.

### Brief Description of the Drawings

[Fig. 1] A plan view showing an iontophoresis device according to an embodiment of the present invention.
[Fig. 2] An enlarged sectional view showing a main portion of each of a working side electrode assembly and a non-working side electrode assembly.
[Fig. 3] Aplan view showing another example of the arrangement of the working side electrode assembly and the non-working side electrode assembly.
[Fig. 4] A plan view showing still another example of the arrangement of the working side electrode assembly and the non-working side electrode assembly.

### Best Mode for carrying out the Invention

Hereinafter, the best mode for carrying out the present invention will be described in detail with reference to the drawings.

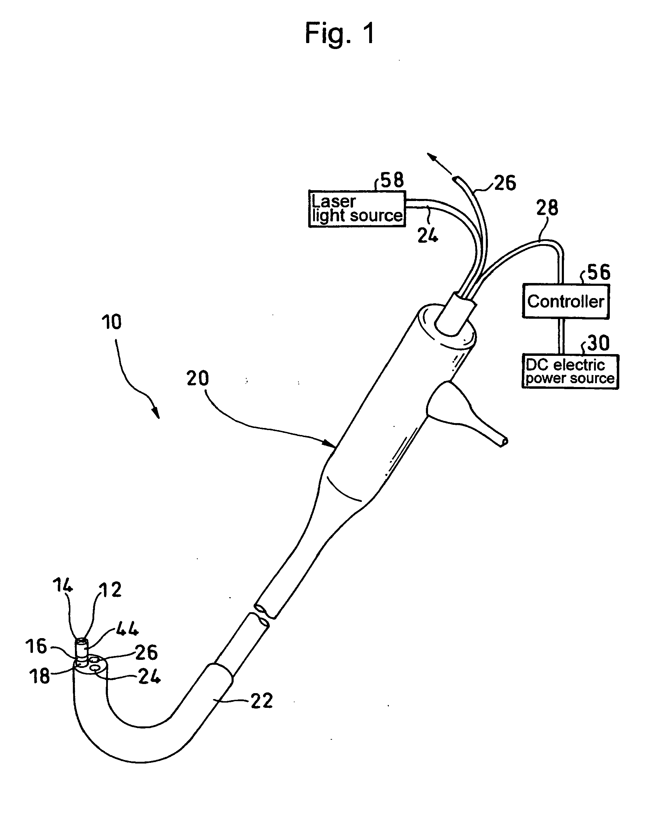

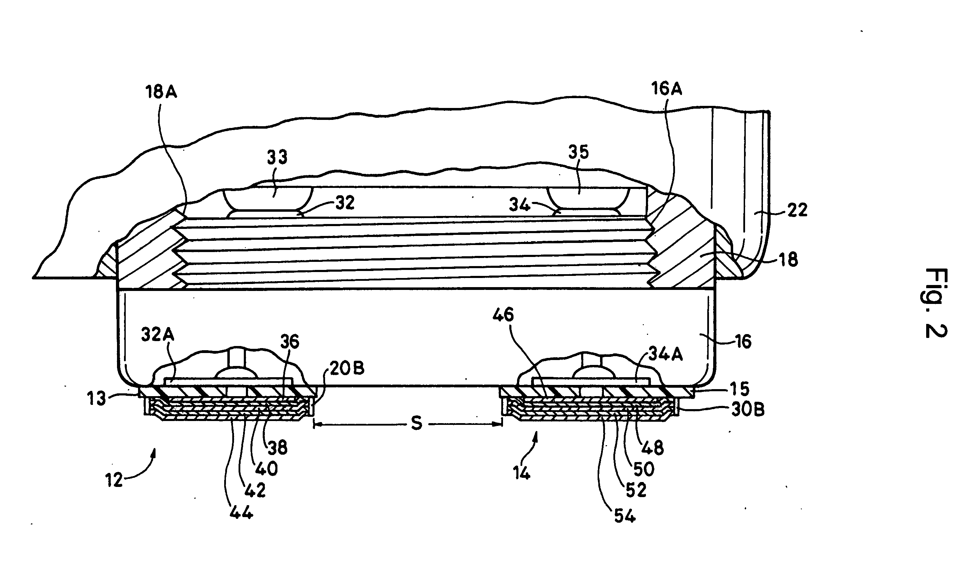

As shown in each of Figs. 1 and 2, a catheter-type iontophoresis device 10 according to the best mode includes: a working side electrode assembly 12 and a non-working side electrode assembly 14 each used for administering an ionic drug; a rod-like member 16 for integrally supporting them; and a DC electric power source 30 connected to the working side electrode assembly 12 and the non-working side electrode assembly 14 with different polarities.

Each of the working side electrode assembly 12 and the non-working side electrode assembly 14 is attached to the tip of the rod-like member 16. The rod-like member 16 is detachably supported by the tip of a flexible cable 18. As a result, the working side electrode assembly 12 and the non-working side electrode assembly 14 are exchangeable integrally with the rod-like member 16.

The flexible cable 18 is supported by a flexible tube 22 of an endoscopic device 20 so that the cable can freely curve. The rod-like member 16 is detachably attached to a tip projecting from the flexible tube 22.

The endoscopic device 20 has an endoscopic optical system including an optical fiber 24 for irradiation light and an optical fiber 26 for reflected light each passing through the flexible tube 22. The optical fiber 24 for irradiation light emits irradiation light from its tip, and the optical fiber 26 for reflected light captures reflected light generated by the irradiation light emitted from the optical fiber 24 for irradiation light at, for example, an affected area in an organism and guides the reflected light to the outside. White light is adapted to be incident from a laser light source 58 to be described later on the optical fiber 24 for irradiation light.

The working electrode assembly 12 and the non-working electrode assembly 14 are connected to different polarities of the DC electric power source 30 via an electric power source circuit (not shown).

The tip of the rod-like member 16 on the side of the flexible cable 18 is provided with a working side electrode terminal 32 to be connected to the working side electrode assembly 12 and a non-working side electrode terminal 34 to be connected to the non-working side electrode assembly 14.

The working side electrode terminal 32 and the non-working side electrode terminal 34 are adapted to be connected to an electric power source side working electrode terminal 33 and an electric power source side non-working electrode terminal 35 on the side of the flexible cable 18, respectively, when the rod-like member 16 is attached to the flexible cable 18.

The electric power source side working electrode terminal 33 and the electric power source side non-working electrode terminal 35 are connected to the DC electric power source 30 arranged further outside the endoscopic device 20 via the electric power source circuit 28.

The rod-like member 16 is a cylindrical member having the same diameter as that of the flexible cable 18. The member 16 is adapted to be attached by threading a male screw portion 16A into a female screw portion 18A at the tip of the flexible cable 18 and to be detached by rotating the male screwportion 16A in a direction opposite to that at the time of threading.

Fig. 2 is an enlarged view showing a state where the working side electrode assembly 12 and the non-working side electrode assembly 14 are arranged so that their central axis lines are parallel to each other. In addition, the working side electrode assembly 12 is constituted by laminating a working side electrode 36, an electrolyte solution holding portion 38, a second ion exchange membrane 40, a drug solution holding portion 42, and a first ion exchange membrane 44 in the stated order from the side of the rod-like member 16, and is formed into a disk shape of about 2 to 6 mm in diameter.

The working side electrode 36 is desirably constituted by a conductive paint applied to the one surface of the base sheet 13 and blended with a nonmetal conductive filler such as a carbon paste. The working side electrode 36 can be constituted by a copper plate or a metal thin film, but a metal eluted from the plate or the thin film may transfer to an organism upon administration of a drug. Therefore, the working electrode 36 is preferably nonmetallic.

The electrolyte solution holding portion 38 is constituted by, for example, an electrolytic paint applied to the working side electrode 36. The electrolytic paint is a paint containing an electrolyte, and an electrolyte that is oxidized or reduced more easily than the electrolytic reaction of water (oxidation on a plus pole and reduction on a minus pole) is particularly preferably used. Examples of such electrolyte include: medical agents such as ascorbic acid (vitamin C) and sodium ascorbate; and organic acids such as lactic acid, oxalic acid, malic acid, succinic acid, and fumaric acid and/or salts thereof. The use of such electrolyte can suppress the generation of an oxygen gas or a hydrogen gas. In addition, blending a plurality of kinds of electrolytes serving as a combination of buffer electrolyte solutions when dissolved in a solvent can suppress a change in pH during energization.

The electrolytic paint is blended with a hydrophilic polymer such as polyvinyl alcohol, polyacrylic acid, polyacrylamide, or polyethylene glycol in order to improve the application property and film-forming property of the paint, and is blended with an appropriate amount of solvent such as water, ethanol, or propanol for adjusting the viscosity of the electrolytic paint. The paint may be blended with an appropriate additional component such as a thickener, a thixotropic agent, a defoaming agent, a pigment, a flavor, or a coloring agent.

The second ion exchange membrane 40 is formed by applying a second ion exchange paint to the electrolyte solution holding portion 38.

The second ion exchange paint is a paint containing an ion exchange resin into which an ion exchange group using, as a counter ion, an ion having a conductivity type opposite to that of a drug ion in the drug solution holding portion 42 to be described later is introduced. In the case where a drug whose drug component dissociates to plus drug ions is used in the drug solution holding portion 42, the paint is blended with an anion exchange resin. On the other hand, in the case where a drug whose drug component dissociates to minus drug ions is used, the paint is blended with a cation exchange resin.

The drug solution holding portion 42 is composed of a drug paint applied to the second ion exchange membrane 40. The paint is a paint containing a drug (including a precursor for the drug) whose drug component dissociates to plus or minus ions (drug ions) as a result of, for example, dissolution into a solvent such as water. Examples of a drug whose drug component dissociates to plus ions can include lidocaine hydrochloride as an anesthetic drug and morphine hydrochloride as an anesthetic drug. Examples of a drug whose drug component dissociates to minus ions can include ascorbic acid as a vitamin agent.

The first ion exchange membrane 44 is formed of a first ion exchange paint applied to the drug solution holding portion 42. The first ion exchange paint is a paint containing an ion exchange resin into which an ion exchange group using, as a counter ion, an ion having the same conductivity type as that of the drug ion in the drug solution holding portion 42 is introduced. In the case where a drug whose drug component dissociates to plus/minus drug ions is used in the drug solution holding portion 42, the paint is blended with an anion/cation exchange resin.

An ion exchange resin obtained by introducing a cation exchange group (an exchange group using a cation as a counter ion) such as a sulfonic group, a carboxylic group, or a phosphoric group into a polymer having a three-dimensional network structure such as a hydrocarbon-based resin (for example, a polystyrene resin or an acrylic resin) or a fluorine-based resin having a perfluorocarbon skeleton can be used as the cation exchange resin without any limitation.

An ion exchange resin obtained by introducing an anion exchange group (an exchange group using an anion as a counter ion) such as a primary amino group, a secondary amino group, a tertiary amino group, a quaternary ammonium group, a pyridyl group, an imidazole group, a quaternary pyridinium group, or a quaternary imidazolium group into a polymer having a three-dimensional network structure similar to that in the case of the cation exchange resin can be used as the anion exchange resin without any limitation.

The non-working side electrode assembly 14 is constituted by laminating a non-working electrode 4 6, a second electrolyte solution holding portion 48, a third ion exchange membrane 50, a third electrolyte solution holding portion 52, and a fourth ion exchange membrane 54 in the stated order on one side of a non-working side base sheet 15, and is formed into a disk shape as in the case of the working side electrode assembly 12.

The non-working side electrode 46 has the same constitution as that of the working side electrode 36 in the working side electrode assembly 12, and the constitutions and components of the second electrolyte solution holding portion 48 and the third electrolyte solution holding portion 52 are the same as those of the electrolyte solution holding portion 38.

The third ion exchange membrane 50 is formed of an ion exchange paint applied to the second electrolyte solution holding portion 48. The ion exchange paint is the same as the first ion exchange paint of which the first ion exchange membrane 44 is formed, and functions as an ion exchange membrane similar to the first ion exchange membrane 44.

The fourth ion exchange membrane 54 is formed of the same second ion exchange paint as that described above applied to the third electrolyte solution holding portion 52. The fourth ion exchange membrane 54 functions as an ion exchange membrane similar to the second ion exchange membrane 40.

A working side electrode terminal plate 32A is arranged on the other surface of the base sheet 13, and conduction is established between the working side electrode terminal plate 32A and the working side electrode 36 of the working side electrode assembly 12 through a through-hole formed on the base sheet 13, and the working side electrode terminal plate 32A is connected to the working side electrode terminal 32 through the through-hole.

Similarly, a non-working side electrode terminal plate 34A is arranged on the other surface of the non-working side base sheet 15, and conduction is established between the non-working side electrode terminal plate 34A and the non-working side electrode 46 of the non-working side electrode assembly 14 through a through-hole formed on the non-working base sheet 15, and the non-working side electrode terminal plate 34A is connected to the non-working side electrode terminal 34 through the through-hole.

The first ion exchange membrane 44 and the fourth ion exchange membrane 54 at the tips of the working side electrode assembly 12 and the non-working side electrode assembly 14 are exposed so as tobe capable of contacting with the side of anorganism, respectively.

The DC electric power source 30 is composed of, for example, an AC/DC converter, and the electric power source circuit 28 between the DC electric power source 30 and the electric power source side working electrode terminal 33 and between the DC electric power source 30 and the electric power source side non-working electrode terminal 35 is provided with a controller 56 for adjusting, out of a current value during energization and an energization time as administration time, at least the current value. As a result, each of the current value and the administration time can be adjusted in a certain range.

Here, a predetermined amount of spacing S is provided between the first ion exchange membrane 44 and the fourth ion exchange membrane 54 at each of the tips of the working side electrode assembly 12 and the non-working side electrode assembly 14 in order to prevent a current from directly flowing between the membranes upon energization. The spacing S has substantially the same size as that of the diameter of each of the first ion exchange membrane 44 and the fourth ion exchange membrane 54.

Upon therapy, the following procedure has only to be performed. White light is applied from the optical fiber 24 for irradiation light. Reflected light (image) is guided to the outside by means of the optical fiber for reflected light 26. An affected area is identified while the reflected light (image) is observed. The working side electrode assembly 12 is pressed against the affected area. No lighting is performed during iontophoresis.

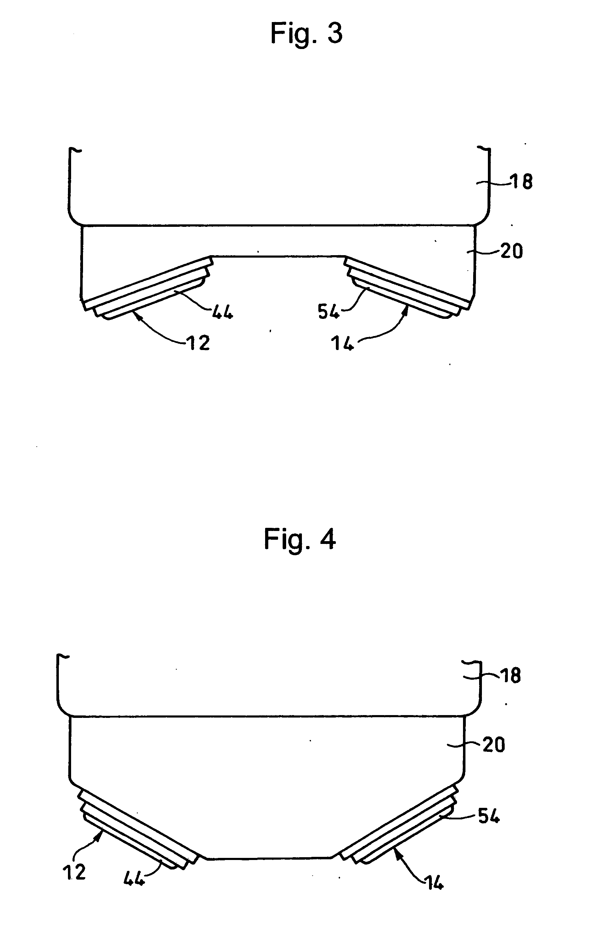

In the above embodiment, the working side electrode assembly 12 and the non-working side electrode assembly 14 are attached such that central axes thereof are in parallel with each other. However, the present invention is not limited thereto. For example, as shown in Fig. 4, the working side electrode assembly 12 and the non-working side electrode assembly 14 may be placed such that central axes thereof intersect each other in a tip direction with an angle of 60° between the axes. Alternatively, as shown in Fig. 4, the working side electrode assembly 12 and the non-working side electrode assembly 14 may be placed such that central axes thereof spread out to a tip direction.

In each of those embodiments, the working side electrode assembly 12 and the non-working side electrode assembly 14 are arranged at the tip of the flexible cable 18 in the endoscopic device 20 with a gap S between the assemblies. Therefore, for example, when a drug solution is caused to permeate into a cancer site of a digestive organ, a doctor grips the endoscopic device 20 to bring the first ion exchange membrane 44 at the tip of the working side electrode assembly 12 at the tip of the flexible cable 18 into close contact with the cancer site and, at the same time, to bring the fourth ion exchange membrane 54 at the tip of the non-working side electrode assembly 14 into close contact with a mucosa or the like near the cancer site for energization. Thus, a target drug solution can be easily caused to permeate into a target site on a pinpoint basis.

In addition, the working side electrode assembly 12 and the non-working side electrode assembly 14 can be detached together with the rod-like member 16 from the flexible cable 18, so a drug solution can be easily exchanged.

The catheter-type iontophoresis device 10 can be used for the therapy of the inside of a body by means of a PDT as an anti-cancer remedy involving: applying a photosensitizing reaction substance to, for example, a cancer cell; and irradiating the substance with light to cause the substance to absorb the light. For example, the device 10 can be used upon therapy of a superficial esophageal cancer, a superficial gastric cancer, or a cerivical cancer. In addition, the device 10 can be used for the therapy of the inside of a body, for example, the therapy of gastric ulcer or colitis by means of a method except a PDT.

In case of a PDT, the drug solution holding portion 42 in the working side electrode assembly 12 holds a photosensitizing reaction substance, and light having a wavelength to be absorbed by the photosensitizing reaction substance such as ultraviolet light is supplied from the laser light source 58 while being controlled with the controller 56 to the optical fiber 24 for irradiation light so that an affected area can be irradiated with the light.

Light having a wavelength to which a photosensitizing reaction substance is sensitive is used for a PDT. In this case, a light source emitting light having the wavelength is separately arranged, and white light and light having the wavelength are selectively switched by using means for switching input to the optical fiber for irradiation light 24 (not shown). In addition, a filter passing only light having the wavelength out of white light may be used without the use of a new light source.

### Industrial Applicability

In the present invention, each of the working electrode assembly and the non-working electrode assembly in the catheter-type iontophoresis device is arranged at the tip of the flexible cable in the endoscopic device. An anti-cancer agent is caused to permeate into a pinpoint such as a cancer site in, for example, a digestive organ so that an efficient therapy can be performed with little side effect. In addition, immediately after a PDT, each of the working electrode assembly and the non-working electrode assembly is exchanged and then an anti-cancer agent is administered. As a result, a therapy and the prevention of recurrence can be simultaneously performed.

## Claims

1. Acatheter-type iontophoresis device comprising a working side electrode assembly and a non-working side electrode assembly each used for administering an ionic drug by iontophoresis and a DC electric power source to be connected to the working side electrode assembly and the non-working side electrode assembly with opposite polarities,
**characterized by** comprising:
a rod-like member for supporting the working side electrode assembly and the non-working side electrode assembly; and
an endoscope device for detachably supporting the rod-like member,
the working side electrode assembly and the non-working side electrode assembly being disposed at a tip of the rod-like member,
a predetermined amount of spacing being provided between the working side electrode assembly and the non-working side electrode assembly, and
the rod-like member being detachably supported at a tip of a flexible cable flexibly supported by a flexible tube of the endoscope device.

2. The catheter-type iontophoresis device according to claim 1, **characterized in that** the ionic drug comprises a photosensitized reactive material to be activated by absorbing light, and the endoscope device comprises an irradiation optical system for applying light from a neighborhood of a tip of the working side electrode assembly via the flexible tube.

3. The catheter-type iontophoresis device according to claim 2, **characterized in that**
the endoscope device comprises an endoscope optical system including an optical fiber for irradiated light for irradiating an inside of an organism with light and an optical fiber for reflected light for introducing reflected irradiated light to an outside, and
the irradiation optical system comprises the optical fiber for irradiated light.

4. The catheter-type iontophoresis device according to any one of claims 1 to 3, **characterized in that**
the flexible cable comprises an electric power source side working electrode terminal and an electric power source side non-working electrode terminal connected via wiring from the DC electric power source to the DC electric power source with opposite polarities the wiring being housed in the flexible cable,
the rod-likemember comprises on a proximal endof a side thereof detachable from the flexible cable a working electrode side contact and a non-working electrode side contact which are connected to or are separated from the electric power source side working electrode terminal and the electric power source side non-working electrode terminal when attached to or detached from the flexible cable, and
the working electrode side contact and the non-working electrode side contact are connected to a working side electrode and a non-working side electrode in the working side electrode assembly and the non-working side electrode assembly, respectively.

5. The catheter-type iontophoresis device according to claim 4, **characterized in that** the endoscope device comprises a controller for adjusting a current value out of a current value during energization and energization time as administration time, the controller being disposed in an electric power source circuit between each of the electric power source side working electrode terminal and the electric power source side non-working electrode terminal and the DC electric power source at least the current value.

6. The catheter-type iontophoresis device according to any one of claims 1 to 5, **characterized in that** the working side electrode assembly and the non-working side electrode assembly are disposed such that central axes thereof are in parallel to each other.

7. The catheter-type iontophoresis device according to any one of claims 1 to 5, **characterized in that** the working side electrode assembly and the non-working side electrode assembly are disposed such that central axes thereof spread in a tip direction.

8. The catheter-type iontophoresis device according to any one of claims 1 to 5, **characterized in that** the working side electrode assembly and the non-working side electrode assembly are disposed such that central axes thereof intersect each other in a tip direction.

9. The catheter-type iontophoresis device according to any one of claims 1 to 8, **characterized in that** the working side electrode assembly comprises:
the working side electrode connected to the DC electric power source having the same polarity as that of a charged ion of the ionic drug;
an electrolyte solution holding portion holding an electrolyte solution, the electrolyte solution holding portion being disposed on a front surface of the working electrode;
a second ion exchange membrane selecting an ion having a polarity opposite to that of the charged ion of the ionic drug, the second ion exchange membrane being disposed on a front surface of the electrolyte solution holding portion;
a drug solution holding portion holding the ionic drug, the drug solution holding portion being disposed on a front surface of the second ion exchange membrane; and
a first ion exchange membrane which is the ion exchange membrane selecting an ion having the same polarity as that of the charged ion of the ionic drug, the first ion exchange membrane being disposed on a front surface of the drug solution holding portion, and the non-working side electrode assembly comprises:
the non-working side electrode connected to the DC electric power source with a polarity opposite to that of the charged ion of the ionic drug;
a second electrolyte solution holding portion holding a second electrolyte solution, the second electrolyte solution holding portion being disposed on a front surface of the non-working side electrode;
a third ion exchange membrane selecting an ion having a polarity same to that of the charged ion of the ionic drug, the third ion exchange membrane being disposed on a front surface of the second electrolyte solution holding portion;
a third electrolyte solution holding portion holding a third electrolyte solution, the third electrolyte solution holding portion being disposed on a front surface of the third ion exchange membrane; and
a fourth ion exchange membrane which is the ion exchange membrane selecting an ion having a polarity opposite to that of the charged ion of the ionic drug, the fourth ion exchange membrane being disposed on a front surface of the third electrolyte solution holding portion.
